# EUROPÄISCHE PATENTSCHRIFT

(11) **EP 1 020 185 B1**
(45) Veröffentlichungstag und Bekanntmachung des Hinweises auf die Patenterteilung: **02.01.2004**
(21) Anmeldenummer: 99125470.7
(22) Anmeldetag: 21.12.1999
(51) Int. Cl.: A61K 31/135, A61P 25/04, A61K 9/52, A61K 9/50

(54) **Opioide Analgetika mit kontrollierter Wirkstofffreisetzung**
Opioid analgesics with controlled release
Opioides analgésiques à libération contrôlée

(30) Priorität: 18.01.1999 DE 19901687
(43) Veröffentlichungstag der Anmeldung: 19.07.2000
(73) Patentinhaber: Grünenthal GmbH, 52078 Aachen (DE)
(72) Erfinder: Betzing, Jürgen, Dr., 79539 Lörrach-Tumringen (DE); Bartholomäus, Johannes, Dr., 52080 Aachen (DE)

(56) Entgegenhaltungen:
- DE-A- 3 306 250
- US-A- 5 095 151
- Y. CHA ET AL.: "a one-week subdermal delivery system for l-methadone based on biodegradable microcapsules" JOURNAL OF CONTROLLED RELEASE, Bd. 7, Nr. 1, April 1988 (1988-04), Seiten 69-78, XP002143620 Amsterdam (NL)
- MUTSCHLER: "Arzneimittelwirkungen", 1991, WISSENSCHAFTLICHE VERLAGSGESELLSCHAFT, STUTTGART
- BURGER A. ET AL: "Hunnius Pharmazeutisches Wörterbuch", 1993, WALTER DE GRUYTER, BERLIN

## Beschreibung

Die vorliegende Erfindung betrifft eine oral zu verabreichende Arzneimittelformulierung, aus der der opioide, analgetische Wirkstoff kontrolliert freigesetzt wird.

Aus dem Stande der Technik sind eine Vielzahl von Formulierungen analgetischer Schmerzmittel bekannt, die eine kontrollierte Freisetzung des Wirkstoffes gewährleisten.

So wird u.a. in der EP-A-0647448 bereits ein analgetisch wirksames Präparat mit einer verzögerten Wirkstoffabgabe beschrieben, das aus einer Vielzahl, in retardierter Form vorliegender, opioidhaltiger Substrate mit einem Durchmesser von 0,1 bis 3 mm als eine Eintagesdosis besteht. Dafür geeignete Substrate können in Form von Sphäroiden, Mikrokugeln, Pellets oder Granulate vorliegen. Um diese Art von Substraten herzustellen, sind in der Regel relativ aufwendige Formulierungstechniken, wie z.B. das Schichtaufbauverfahren für Pellets oder das Extrusions-, Sphäronisationsverfahren für Sphäroide notwendig.

Andererseits fallen viele opioide Wirkstoffe bei ihrer Herstellung in kristalliner Form an, so daß ein Bedarf entsteht, diese unmittelbar, d.h. ohne die genannten, aufwendigen Formulierungstechniken bei der Arzneimittelherstellung einzusetzen.

Aus dem Journal of Controlled Release 7 (1988), Seite 69-78 sind subdermal zu verabreichende Mikrokapseln bekannt, die den Wirkstoff L-Methadon in Form von Kristallen aufweisen können.

Aufgabe der vorliegenden Erfindung war es daher, ein oral zu verabreichendes Präparat mit einer kontrollierten Freisetzung wenigstens eines opioiden Wirkstoffes zur Verfügung zu stellen, bei dem die bei der Wirkstoffherstellung erhaltenen Kristalle unmittelbar, d.h. ohne aufwendige Formulierungsschritte eingesetzt werden können.

Erfindungsgemäß wird diese Aufgabe gelöst durch Bereitstellung eines oral zu verabreichenden Präparates mit einer kontrollierten Freisetzung eines opioiden Analgetikums in Form von Kristallen mit einer Partikelgröße von 10 µm bis 3 mm, die wenigstens einen retardierenden Überzug aufweisen.

Vorzugsweise haben die Kristalle eine Partikelgröße von 50 µm bis 1 mm. Als analgetischen Wirkstoff enthalten die erfindungsgemäßen Präparate wenigstens ein Opioid in kristalliner Form. Als Opioide können Hydromorphon, Oxycodon, Morphin, Levorphanol, Methadon, Dihydrocodein, Codein, Dihydromorphin, Pethidin, Fentanyl, Piritramid, Buprenorphin, Tilidin, Tramadol, deren jeweilige Salze oder deren Mischungen eingesetzt werden.

Ganz besonders bevorzugt wird als Analgetikum Tramadol, Tramadol-Hydrochlorid, Morphin, Morphin-Hydrochlorid und/oder Morphin-Sulfat verwendet.

Die Wirkstoff-Kristalle der erfindungsgemäßen Präparate können Einkristalle sein oder einen polykristallinen Aufbau aufweisen.

Neben den genannten opioiden Analgetika können nicht opioide Analgetika in dem erfindungsgemäßen Präparat vorhanden sein, die mit den opioiden Analgetika gegebenenfalls eine synergistische Wirkung zeigen. Zu diesen nicht opioiden Analgetika gehören Ibuprofen, Ketoprofen, Flurbiprofen, Propyphenazon, Paracetamol, Naproxen, Acemetacin, Acetylsalicylsäure, Metamizol und deren Salze, vorzugsweise in Form von Kristallen.

Die erfindungsgemäß zum Einsatz kommenden Präparate zeichnen sich durch eine kontrollierte, vorzugsweise verzögerte Freisetzung des Analgetikums aus.

Dies wird erreicht, indem die Wirkstoffkristalle mit zumindest einem retardierenden Überzug versehen werden. Durch diesen Überzug wird erreicht, daß eine kontrolliert verzögerte Freisetzung des Wirkstoffes über den gewünschten Zeitraum bewirkt wird. Dadurch gelingt es, die Wirkungsdauer gegenüber herkömmlichen Darreichungsformen, d. h. solchen ohne retardierenden Überzug, gezielt zu steuern. Vorzugsweise wird dabei angestrebt, die Freigabe des Wirkstoffes so einzustellen, daß das Präparat höchstens zweimal, vorzugsweise nur einmal täglich verabreicht werden muß.

Als Überzugsmaterialien sind alle pharmazeutisch unbedenklichen Überzugsmaterialien geeignet, die dem Fachmann bekannt sind. Vorzugsweise werden natürliche, gegebenenfalls modifizierte oder synthetische Polymere als Überzugsmaterialien verwendet. Es sind dies Polymere, wie z. B. Celluloseether oder Acrylharze. Ganz besonders bevorzugt sind wasserunlösliche oder wasserquellbare Cellulosederivate, wie Alkylcellulose, vorzugsweise Ethylcellulose, oder wasserunlösliche Acrylharze, wie Poly(meth)acrylsäure und/oder deren Derivate, wie deren Salze, Amide oder Ester. Es können auch wasserunlösliche Wachse als Überzugsmaterial verwendet werden.

Diese Materialien sind aus dem Stande der Technik, z. B. Bauer, Lehmann, Osterwald, Rothgang "Überzogene Arzneiformen", Wissenschaftliche Verlagsgesellschaft mbH Stuttgart, 1998, Seite 69 ff., bekannt und werden hiermit als Referenz eingeführt.

Neben den wasserunlöslichen Polymeren und Wachsen können gegebenenfalls zur Einstellung der Freisetzungsrate des Wirkstoffes auch vorzugsweise bis zu 30 Gew.% an nicht retardierenden, vorzugsweise wasserlöslichen Polymeren, wie beispielsweise Polyvinylpyrrolidon oder wasserlösliche Cellulosederivate, wie Hydroxyethylcellulose, Hydroxypropylmethylcellulose oder Hydroxyprop ylcellulose, und/oder bekannte Weichmacher mitverwendet werden.

Neben dem retardierten Überzug können die Wirkstoff-Kristalle noch mit weiteren Überzügen ausgerüstet werden. Dabei kann beispielsweise ein solcher Überzug aus einem vom Material des retardierenden Überzug unterschiedlichen Material als nichtretardierende Trennschicht auf der Kristalloberfläche aufgebracht sein.

Als Überzugsmaterialien kommen für diese Trennschicht bevorzugt Celluloseether, Polyvidone, Polyacrylate oder auch polymere Naturstoffe in Frage.

Es ist auch möglich, den weiteren Überzug, vorzugsweise über den retardierenden Überzug, aus dem Wirkstoff der Kristalle oder aus einem von diesen unterschiedlichen vorzugsweise opioiden Analgetikum zu machen, aus dem dieser Wirkstoff unretardiert nach der oralen Verabreichung freigesetzt wird. Durch diesen Mehrschichtenüberzug kann nach der Verabreichung des Präparates sehr rasch eine Initialdosis des Analgetikums zur anfänglichen Schmerzlinderung zur Verfügung gestellt werden, wobei das Niveau des Analgetikums durch die anschließende retardierte Abgabe des Wirkstoffes gehalten werden kann. Als Überzugsmaterialien kommen dafür pharmazeutisch unbedenkliche Materialien in Kombination mit dem Initialwirkstoff, wie beispielsweise Celluloseether, Polyvidone oder Polyacrylate in Frage. Es ist aber auch möglich, in dem unretardierenden Überzug zu oder anstelle des Wirkstoffes der Kristalle oder des von diesem unterschiedlichen, weiteren vorzugsweise opioiden Analgetikums einen anderen pharmazeutischen Wirkstoff vorzusehen.

Desweiteren können die Kristalle neben dem retardierenden Überzug auch noch Überzüge aufweisen, die sich pHabhängig auflösen. So kann z. B. erreicht werden, daß zumindest ein Teil der Kristalle eines Präparates den Magentrakt unaufgelöst passiert und erst im Darmtrakt freigesetzt wird.

Eine weitere bevorzugte Ausführungsform der erfindungsgemäßen Präparate besteht darin, daß sie neben den mit einem retardierenden und gegebenenfalls weiteren Überzügen ausgerüsteten Wirkstoffkristalle, die eine kontrollierte Freisetzung des Wirkstoffes garantieren, auch unretardierte, aber mit einem oder mehreren der genannten Nichtretard-Überzüge aufweisende Wirkstoffkristalle enthalten.

Die Herstellung der opioiden Wirkstoffkristalle ist bekannt. Dabei fallen die Kristalle unmittelbar bei der notwendigen Reinigung des Wirkstoffes an.

Die unmittelbar bei der Herstellung, vorzugsweise nach dem Umkristallisieren erhaltenen Wirkstoffkristalle werden mit Überzügen nach üblichen, bekannten Verfahren, wie durch z. B. Aufsprühen von Lösungen, Dispersionen und/oder Emulsionen oder durch Pulverauftragverfahren versehen. Auch die Koazervation stellt ein geeignetes Verfahren dar.

Vorzugsweise wird hierzu zunächst eine Trennschicht über die einzelnen Kristalle aufgebracht, indem die Wirkstoffkristalle nach dem letzten Reinigungsschritt, der Kristallisation und Trocknung, durch Aufsprühen einer Lacklösung oder vorzugsweise einer wässrigen Überzugsdispersion überzogen werden. Darüber wird der retardierende Überzug wieder durch Aufsprühen einer Überzugsdispersion und anschließendem Trocknen aufgebracht. Die Dicke dieses Überzugs kann gemäß dem zu erzielenden Freisetzungsprofil variiert werden.

Sofern noch weitere Überzüge aufgebracht werden, werden diese vorzugsweise nach derselben Methode hergestellt.

Ein weiterer Gegenstand der Erfindung ist die Bereitstellung der erfindungsgemäßen, oral zu verabreichenden Präparate in Form von Kapseln, in denen die Wirkstoffkristalle mit einer kontrollierten Freisetzung des opioiden Analgetikums entsprechend der individuell zu erreichenden Freisetzungsdauer und freizusetzenden Menge an Analgetikum vorhanden sind. Vorzugsweise wird die Menge an Wirkstoffkristallen in einer Kapsel so gewählt, daß die Dosis für eine zweimalige, vorzugsweise einmalige Applikation pro Tag ausreicht. Diese Retardformulierung in Kapseln erfolgt ebenso ohne aufwendige Formulierungstechnologie, da die überzogenen Wirkstoffkristalle nur in Kapseln abzufüllen sind. Die Wirkstoffkristalle können erfindungsgemäß auch in einer Flasche oder einem Sachet zu den Dosiseinheiten zusammengefaßt sein oder mittels eines Spenders volumetrisch dosiert werden. Ein Zusatz üblicher Hilfsstoffe wie Füllmittel, Gleitmittel oder Zerfallsförderer ist möglich.

Darüber hinaus können die erfindungsgemäßen, oral zu verabreichenden Präparate auch in Form einer Tablette vorliegen, zu der die überzogenen Wirkstoffkristalle entsprechend der individuell zu erreichenden Freisetzungsdauer und freizusetzenden Menge an opioidem Analgetikum mit oder ohne Zugabe von übliche Tabletten-Hilfs- und Zusatzstoffen zu einer Tablette verpreßt werden. Auch hier ist es vorteilhaft, wenn die Menge der Wirkstoffkristalle, aus der sich die Tablette zusammensetzt, so gewählt wird, daß die zu erreichende Freisetzungsdauer und freizusetzende Menge an Analgetikum für eine zweimalige, vorzugsweise einmalige Applikation pro Tag ausreicht.

Vorzugsweise werden Tabletten mit hohem Anteil an Hilfsstoffen hergestellt, damit in die überzogenen Wirkstoff-Kristalle individuell erhalten bleiben. Beim schnellen Zerfall der Tabletten werden dann die Kristalle, aus denen die retardierte Freisetzung erfolgt, freigegeben. Auch beim Teilen einer Tablette bleibt das Freisetzungsprofil der retardierten Wirkstoffkristalle erhalten.

Es ist auch möglich, Tabletten mit niedrigem Anteil an Hilfstoffen herzustellen. Dabei können die überzogenen Wirkstoff-Kristalle beim Verpressen miteinander aggregieren und so eine zusätzliche Retardmatrix bilden. Solche Tabletten zerfallen nicht mehr spontan, so daß die Retardierung gegenüber individuell überzogenen Kristalle ausgeprägter ist.

Vorzugsweise weisen die erfindungsgemäßen Präparate eine Gesamtkonzentration an Tramadol, als Hydrochlorid-Salz berechnet, von 10 bis 1000 mg, vorzugsweise 50 bis 800 mg, auf.

### Beispiele

### Beispiel 1

Es wurden Tramadolhydrochloridkristalle mit polykristallinem Aufbau in einer Partikelgröße von 250 bis 500 µm nach ihrer Herstellung und Reinigung ohne weiteren Formulierungsschritt eingesetzt. Die Angaben zur Menge der Ethylcellulose beziehen sich auf die Trockenmasse nach Aufbringen der wässrigen Dispersion.

### Zusammensetzung:

| | | |
|---|---|---|
| | | |
| Kristalle aus | Tramadol HCl | 1,000 kg |
| | | |
| Überzug aus | Ethylcellulose (Aquacoat®) | 0,200 kg |
| | Dibutylsebacat | 0,050 kg |
| | | |
| Total | | 1,250 kg |

Diese Tramadolhydrochloridkristalle wurden in einem Wirbelschichtgerät mittels erwärmter Luft bewegt, die wässrige Ethylcellulose-Suspension, in die Dibutylsebacat vorher eingerührt worden war, wurde mittels einer Zweistoffdüse auf die Kristalle langsam aufgesprüht. Nach Aufbringen der Suspension wurden die Kristalle getrocknet.

### Beispiel 2

Die gemäß Beispiel 1 überzogenen Kristalle wurden zu Kapseln verarbeitet.

Dazu wurden die überzogenen Kristalle in einem Kubusmischer mit den oben aufgeführten Hilfsstoffen gemischt und mittels einer Kapselmaschine in Hartgelatinekapseln der Größe 2 abgefüllt.

### Zusammensetzung:

| Kapselfüllung | pro Kapsel | pro Ansatz |
|---|---|---|
| Tramadol HCl Kristalle, überzogen gemäß Beispiel 1 | 125 mg | 1,25 kg |
| Mikrokristalline Cellulose | 75 mg | 0,75 kg |
| Natriumcarboxymethylstärke, Typ A | 45 mg | 0,45 kg |
| Magnesiumstearat | 5 mg | 0,05 kg |
| | | |
| Total | 250 mg | 2,50 kg |

### Beispiel 3

250 g der gemäß Beispiel 1 überzogenenKristalle wurden mit 344 g mikrokristalliner Cellulose und 6 g Magnesiumstearat gemischt und zu schnell zerfallenden Tabletten mit einem 10 mm Durchmesser und 300 mg Gewicht verpreßt .

### Beispiel 4

Es wurden Tramadolhydrochloridkristalle mit polykristallinem Aufbau mit einer Partikelgröße von 250 bis 500 µm nach ihrer Herstellung und Reinigung eingesetzt. Die Angaben zur Zwischenschicht wie zur Retardschicht beziehen sich auf die Trockenmasse nach Aufbringen der wässrigen Dispersion.

Die Tramadolhydrochloridkristalle wurden in einem Wirbelschichtgerät mittels erwärmter Luft bewegt und zunächst die wässrige Dispersion zur Herstellung der Zwischenschicht aufgesprüht und getrocknet. Dann wurde die wässrige Ethylcellulose-Suspension, in die Dibutylsebacat vorher eingerührt worden war, mittels einer Zweistoffdüse auf die Kristalle langsam aufgesprüht. Nach Aufbringen der Suspension wurden die Kristalle getrocknet.

### Zusammensetzung:

| | | |
|---|---|---|
| | | |
| Kristalle aus | Tramadol HCl | 1,000 kg |
| | | |
| Zwischenschicht | Macrogol 6000 | 0,025 kg |
| | Talkum | 0,040 kg |
| | Hydroxypropylmethylcellulose Typ 2910, 6 mPas | 0,100 kg |
| | | |
| Überzug aus | Ethylcellulose (Aquacoat®) | 0,200 kg |
| | Dibutylsebacat | 0,050 kg |

## Patentansprüche

1. Ein oral zu verabreichendes Präparat mit einer kontrollierten Freisetzung eines opioiden Analgetikums in Form von Kristallen mit einer Partikelgröße von 10 µm bis 3 mm, die wenigstens einen retardierenden Überzug auf Basis von Polymeren aufweisen, wobei als Polymere Acrylharze und/oder Celiulosederivate und/oder Wachse vorliegen.

2. Präparat nach Anspruch 1, **dadurch gekennzeichnet, daß** die Partikelgröße 50 µm bis 1 mm beträgt.

3. Präparat nach Anspruch 1 oder 2, **dadurch gekennzeichnet, daß** die Kristalle Einkristalle sind oder einen polykristallinen Aufbau aufweisen.

4. Präparat nach einem der Ansprüche 1 bis 3, **dadurch gekennzeichnet, daß** der retardierende Überzug bis zu 30 Gew.-% eines nichtretardierenden Polymeren enthält.

5. Präparat nach einem der Ansprüche 1 bis 4, **dadurch gekennzeichnet, daß** als Cellulosederivat Alkylcellulose eingesetzt wird.

6. Präparat nach einem der Ansprüche 3 bis 5, **dadurch gekennzeichnet, daß** als Polymere Ethylcellulose und/oder Poly(meth)acrylsäure und/oder deren Derivate und gegebenenfalls bis zu 30 Gew.% Hydroxypropylcellulose, Hydroxymethylcellulose, Hydroxypropylmethylcellulose oder Polyvidon eingesetzt werden.

7. Präparat nach einem der Ansprüche 1 bis 6, **dadurch gekennzeichnet, daß** als opioides Analgetikum Hydromorphon, Oxycodon, Morphin, Levorphanol, Methadon, Dihydrocodein, Codein, Dihydromorphin, Pethidin, Piritramid, Fentanyl, Tilidin, Buprenorphin, Tramadol, deren jeweilige Salze oder deren Mischungen eingesetzt werden.

8. Präparat nach Anspruch 7, **dadurch gekennzeichnet, daß** das opioide Analgetikum Tramadol, Tramadol-Hydrochlorid und/oder Morphin, Morphin-Hydrochlorid und/oder Morphin-Sulfat ist.

9. Präparat nach einem der Ansprüche 1 bis 8, **dadurch gekennzeichnet, daß** die Kristalle neben dem retardierenden Überzug mindestens noch einen weiteren Überzug aufweisen.

10. Präparat nach Anspruch 9, **dadurch gekennzeichnet, daß** als weiterer Überzug ein Überzug aus einem vom retardiertenden Überzug unterschiedlichen Material als nichtretardierende Trennschicht direkt auf der Kristalloberfläche aufgebracht ist.

11. Präparat nach Anspruch 9 oder 10, **dadurch gekennzeichnet, daß** als weiterer Überzug ein Magensaft resistenter Überzug vorliegt.

12. Präparat nach einem der Ansprüche 9 bis 11, **dadurch gekennzeichnet, daß** als weiterer Überzug ein Überzug enthaltend denselben oder ein vom Analgetikum der Kristalle unterschiedliches, opioides Analgetikum oder einen anderen pharmazeutischen Wirkstoff vorliegt.

13. Präparat nach den Ansprüchen 1 bis 12, **dadurch gekennzeichnet, daß** die Kristalle in einer Kapsel, einem Sachet, einer Flasche, einem Spender, vorzugsweise einem Dosierspender, vorliegen.

14. Präparat nach den Ansprüchen 1 bis 12, **dadurch gekennzeichnet, daß** die Kristalle mit üblichen Hilfsund Zusatzstoffen zu Tabletten verpreßt sind.

15. Präparat nach den Ansprüchen 1 bis 12, **dadurch gekennzeichnet, daß** die Kristalle gegebenenfalls mit nichtretardierten pharmazeutischen Wirkstoffen ohne Hilfs- und Zusatzstoffe zu Tabletten verpreßt sind.

16. Präparat nach einem der Ansprüche 1 bis 15, **dadurch gekennzeichnet, daß** die Gesamtkonzentration des Tramadols, als Hydrochlorid-Salz berechnet, 10 bis 1000 mg beträgt.

## Claims

1. A preparation to be administered orally with a controlled release of an opioid analgesic in the form of crystals with a particle size of 10 µm to 3 mm which have at least one action-sustaining coating based on polymers, wherein acrylic resins and/or cellulose derivatives and/or waxes are present as the polymers.

2. Preparation according to claim 1, **characterized in that** the particle size is 50 µm to 1 mm.

3. Preparation according to claim 1 or 2, **characterized in that** the crystals are monocrystals or have a polycrystalline structure.

4. Preparation according to one of claims 1 to 3, **characterized in that** the action-sustaining coating comprises up to 30 wt.% of a polymer which is not action-sustaining.

5. Preparation according to one of claims 1 to 4, **characterized in that** alkylcellulose is employed as the cellulose derivative.

6. Preparation according to one of claims 3 to 5, **characterized in that** ethylcellulose and/or poly(meth)acrylic acid and/or derivatives thereof and optionally up to 30 wt.% hydroxypropylcellulose, hydroxymethylcellulose, hydroxypropylmethylcellulose or polyvidone are employed as the polymer.

7. Preparation according to one of claims 1 to 6, **characterized in that** hydromorphone, oxycodone, morphine, levorphanol, methadone, dihydrocodeine, codeine, dihydromorphine, pethidine, piritramide, fentanyl, tilidine, buprenorphine, tramadol, their particular salts or mixtures thereof are employed as the opioid analgesic.

8. Preparation according to claim 7, **characterized in that** the opioid analgesic is tramadol, tramadol hydrochloride and/or morphine, morphine hydrochloride and/or morphine sulfate.

9. Preparation according to one of claims 1 to 8, **characterized in that** in addition to the action-sustaining coating, the crystals have at least one more further coating.

10. Preparation according to claim 9, **characterized in that** as a further coating a coating of a material which differs from the action-sustaining coating is applied directly to the crystal surface as a separating layer which is not action-sustaining.

11. Preparation according to claim 9 or 10, **characterized in that** a coating which is resistant to gastric juice is present as a further coating.

12. Preparation according to one of claims 9 to 11, **characterized in that** a coating comprising the same opioid analgesic as or one which differs from the analgesic of the crystals or another pharmaceutical active compound is present as a further coating.

13. Preparation according to claims 1 to 12, **characterized in that** the crystals are present in a capsule, a sachet, a bottle, a dispenser, preferably a dosed dispenser.

14. Preparation according to claims 1 to 12, **characterized in that** the crystals are pressed to tablets with conventional auxiliary substances and additives.

15. Preparation according to claims 1 to 12, **characterized in that** the crystals are pressed to tablets optionally with pharmaceutical active compounds which are not sustained-action, without auxiliary substances and additives.

16. Preparation according to one of claims 1 to 15, **characterized in that** the total concentration of the tramadol, calculated as the hydrochloride salt, is 10 to 1,000 mg.

## Revendications

1. Préparation à administrer par voie orale, à libération contrôlée d'un analgésique opioïde sous forme de cristaux en particules ayant un diamètre de 10 µm à 3 mm, qui présentent au moins un revêtement retardateur à base de polymères, les polymères présents étant des résines acryliques et/ou des dérivés de cellulose et/ou des cires.

2. Préparation suivant la revendication 1, **caractérisée en ce que** le diamètre des particules va de 50 µm à 1 mm.

3. Préparation suivant la revendication 1 ou 2, **caractérisée en ce que** les cristaux sont des monocristaux ou présentent une structure polycristalline.

4. Préparation suivant l'une des revendications 1 à 3, **caractérisée en ce que** le revêtement retardateur contient jusqu'à 30 % en poids d'un polymère non retardateur.

5. Préparation suivant l'une des revendications 1 à 4, **caractérisée en ce qu'**on utilise une alkylcellulose comme dérivé de cellulose.

6. Préparation suivant l'une des revendications 3 à 5, **caractérisée en ce qu'**on utilise comme polymères de l'éthylcellulose et/ou un polymère d'acide acrylique ou méthacrylique et/ou leurs dérivés et, le cas échéant, jusqu'à 30 % en poids d'hydroxypropylcellulose, d'hydroxyméthylcellulose, d'hydroxypropylméthylcellulose ou de polyvidone.

7. Préparation suivant l'une des revendications 1 à 6, **caractérisée en ce qu'**on utilise comme analgésique opioïde l'hydromorphone, l'oxycodone, la morphine, le lévorphanol, la méthadone, la dihydrocodéine, la codéine, la dihydromorphine, la péthidine, le piritramide, le fentanyle, la tilidine, la buprénorphine, le tramadol, leurs sels correspondants ou leurs mélanges.

8. Préparation suivant la revendication 7, **caractérisée en ce que** l'analgésique opioïde est le tramadol, le chlorhydrate de tramadol et/ou la morphine, le chlorhydrate de morphine et/ou le sulfate de morphine.

9. Préparation suivant l'une des revendications 1 à 8, **caractérisée en ce que** les cristaux portent, outre le revêtement retardateur, au moins encore un autre revêtement.

10. Préparation suivant la revendication 9, **caractérisée en ce qu'**un revêtement d'une matière différente du revêtement retardateur est appliqué directement à la surface des cristaux comme autre revêtement formant une couche de séparation non retardatrice.

11. Préparation suivant la revendication 9 ou 10, **caractérisée en ce qu'**un revêtement résistant au suc gastrique est présent comme autre revêtement.

12. Préparation suivant l'une des revendications 9 à 11, **caractérisée en ce qu'**un revêtement contenant le même analgésique opioïde ou un analgésique opioïde différent de l'analgésique des cristaux ou une autre substance pharmaceutique active est présent comme autre revêtement.

13. Préparation suivant les revendications 1 à 12, **caractérisée en ce que** les cristaux sont présents dans une gélule, un sachet, un flacon, un distributeur, de préférence un distributeur-doseur.

14. Préparation suivant les revendications 1 à 12, **caractérisée en ce que** les cristaux sont pressés en comprimés avec des substances auxiliaires et des additifs classiques.

15. Préparation suivant les revendications 1 à 12, **caractérisée en ce que** les cristaux sont pressés en comprimés, éventuellement avec des substances pharmaceutiques actives non retardées, sans substances auxiliaires ni additifs.

16. Préparation suivant l'une des revendications 1 à 15, **caractérisée en ce que** la concentration totale en tramadol, exprimée en chlorhydrate, est de 10 à 1000 mg.
